# EUROPEAN PATENT SPECIFICATION

(11) **EP 1 901 698 B1**
(45) Date of publication and mention of the grant of the patent: **21.10.2009**
(21) Application number: 06754350.4
(22) Date of filing: 14.06.2006
(51) Int. Cl.: A61K 36/76

(54) **PROCESS FOR EXTRACTING SALICIN DERIVATIVES FROM SALIX**
VERFAHREN ZUR EXTRAKTION VON SALICINDERIVATEN AUS SALIX
PROCÉDÉ POUR L'EXTRAKTION DE DÉRIVÉS DE SALICINE À PARTIR DE SALIX

(30) Priority: 14.07.2005 IT MI20051349
(43) Date of publication of application: 26.03.2008
(73) Proprietor: Indena S.p.A., 20132 Milano (IT)
(72) Inventor: BOMBARDELLI, Ezio, I-27027 Groppello Cairoli (IT); GIORI, Andrea, I-20139 Milano (IT); ANELLI, Alessandro, I-20139 Milano (IT)
(74) Representative: Minoja, Fabrizio
(86) International application number: PCT/EP2006/005703
(87) International publication number: WO 2007/006384

(56) References cited:
- WO-A-02/100423
- WO-A-20/05002611
- DU ET AL: "Semi-industrial isolation of salicin and amygdalin from plant extracts using slow rotary counter-current chromatography" JOURNAL OF CHROMATOGRAPHY A, vol. 1074, May 2005 (2005-05), pages 43-46, XP004867148

## Description

### Summary of the Invention

The present invention relates to a process for extracting salicin derivatives from Salix spp. comprising purification on an adsorption resin column.

The extract is characterized by a high content in salicin derivatives, a reduced content in high molecular tannins and a content in proanthocyanidins sufficient to inhibit some tissue metal proteases. The product can be formulated in oils rich in ω-3 and ω-6 acids which provide a better absorption of the active principles, also increasing synergistically their action.

### Technological background

Bark and branches extracts of different species of *Salix* have been used for unmemorable time for the treatment of articular rheumatic forms and gout. *Salix* extracts were, however, substantially abandoned at the end of the 19^{th} century, when aspirin was synthesized by acetylation of salicylic acid, obtained by oxidation of the compounds present in *Salix*. However, aspirin and *Salix* extracts have substantial differences in terms of mechanisms of action and activity on bone joints. The extracts act on the enzyme COX 2, while aspirin mainly acts on COX 1, which involves the well-known side effects on the gastrointestinal tract and blood coagulation, which severely restrict its prolonged use which is conversely necessary in the case of such chronic-degenerative pathologies as arthrosis and rheumatoid arthritis.

It is known from the literature that *Salix* extracts have extremely variable contents in salicin derivatives, which are on the average up to 15%, and tannin content ranging from 8 to 20%. Tannins present in *Salix* extracts, as is the case with all gallic and catechic tannins, have strong affinity with proteins and proteoglycans, which involves tissue damages in case of long-lasting treatments.

There is therefore the need for a convenient process, which is easily applicable in the industry and provides extracts with standardized contents in the active components.

WO 2005/002611 discloses formulations for the treatment of arthritis comprising saligenin or saligenin containing extracts in combination with other active ingredients.

### Disclosure of the invention

The present invention relates to a process for the preparation of novel *Salix* spp extracts, characterized by an high content in salicin derivatives, a reduced content in high molecular tannins and a sufficient content in proanthocyanidins in order to inhibit some tissue metal proteases.

It has surprisingly been found that the extraction of *Salix* bark or branches under suitable conditions, and the specific purification treatment of the resulting extract, provides extracts with salicin derivatives content up to 50%, tannin content not above 5% and oligomeric procyanindins content higher than 5%.

The advantage of using *Salix* extracts, particularly the extracts prepared by the present invention, compared with salicin derivatives alone, is connected to the presence of proanthocyanidins, strong radicals scavengers and powerful inhibitors of metal proteases, which are activated in arthritic conditions via over-expression of leukocyte Il₁.

The process of the invention for the preparation of *Salix* extracts differs from those of the prior art in the extract contents in salicin and its derivatives and in the use of matrices which provide the selective reduction in tannin contents, while retaining the therapeutically useful proanthocyanidins in the extract.

The process of the invention includes four main steps:
a) Extraction of *Salix* spp branches and bark with suitable solvents which solubilize the desired products (total extract);
b) Removal of water-insoluble (or poorly soluble) tannins;
c) Optional removal of water-soluble tannins;
d) Purification on an adsorption resin colum, to increase the content of salicin derivatives

Step (a) is accomplished by extraction of the vegetable material, consisting of plant bark and branches, with a C1-C3 alcohol or acetone or mixtures of these solvents or aqueous solutions of these solvents or water alone. A 30% v/v water-ethanol solution is preferred.

The extraction temperature can range from 10°C to 80°C, preferably is 25°C.

Step (b) allows to remove water-insolubles, particularly high molecular tannins, from the extract.

Step (c) allows to remove most water-soluble tannins from the extract. This is an optional step, that can be carried out to remove any tannins still present in the extract after step (b). These metabolites can be removed by using polyvinylpolypyrrolidone (PVPP).

Step (d) allows to fractionate the extract removing most useless metabolites (sugars, and the like), while keeping the desired secondary ones, i.e. salicin derivatives and oligomeric proanthocyanidins. This step consist in a chromatographic separation through adsorption on a polymeric resin. Examples of suitable resins for this purpose are Styrene-DVB resins such as AmberliteHP20^{®} or Rohm and Haas XAD1180^{®}, and acrylic resins such as Rohm and Haas XAD7HP^{®}.

During the column fractionation step using suitable solvent mixtures, free salicin can be separated from its derivatives, obtaining fractions rich in free salicin with reduced amounts of its derivatives and fractions with completely different compositions.

The total extract obtained from *Salix* bark and branches with 30% ethanol is concentrated to a dry residue ranging from 5% to 50% w/w, preferably 25% w/w, and left at a temperature from 1°C to 20°C, preferably at 4°C, without stirring for a time from 1 hour to 24 hours, preferably 16 hours.

The resulting suspension is centrifuged at 4°C to remove the residual precipitate containing high molecular derivatives and tannins from the clear aqueous solution.

Water- insoluble or poorly soluble tannins contained in the total extract are removed by water purification, which can be further improved by optional treatment (step c) with polyvinylpolypyrrolidone (PVPP).The partial water purification (step b) can only remove part of tannins (above 50% w/w. of tannins present), while PVPP purification removes residual tannins within values below 5% of the final extract weight.

Therefore, the clear aqueous solution from step b) is treated with PVPP (1-50% w/w, preferably 1:30, most preferably 1:20 on the dry residue of the aqueous extract to treat) keeping stirring for 1 or more hours.

The solution is filtered from PVPP and adsorbed tannins.Then the aqueous solution is adsorbed on the resin, thoroughly washing the substrate with water to remove undesired soluble substances. The solution unretained is discarded.

The product is eluted with a water-alcohol solution (C1-C3 alcohols, preferably ethanol), with water content ranging from 50% v/v to 0% v/v, preferably 10% v/v. Alternatively, a water-acetone solution with water content ranging from 50% v/v to 0% v/v, preferably 10% v/v, can also be used. The water-ethanol solution is concentrated to dryness or atomized. The resulting extract can be formulated in the ordinary pharmaceutical solid forms or as an oily suspension in capsules, particularly in oils rich in ω-3 / ω-6 poly-unsaturated fatty acids; particularly preferred are *Enothera biennis* oil and fish oil and its derivatives.

Active dosages for the treatment of arthrosis and rheumatoid arthritis in humans range from 100 to 1000 mg daily, according to the severity of the disease to treat.

The invention is described in greater detail in the following examples.

### Example 1

### Extraction of Salix branches and bark with a water-ethanol solution (step a):

In this step, the total extract which can be used as the starting material for the subsequent column chromatography separation is prepared.

1000 grams of *Salix* branches and bark are covered with 1.5 liters of 30% v/v ethanol at 20°C for 4 hours in a static percolator. After 4 hours, the percolate is recovered and extracted 6 times again under the same conditions, but using 1 liter of solvent per extraction, to obtain approx. 7 liters of total percolate. The combined percolates are filtered to remove impurities and vegetable residues. This solution (product 1) has a total dry residue of 154 grams, the yield vs. starting material being 15.4% w/w.

The free salicin HPLC content is 4.63%; the total salicin HPLC content is 15.4% w/w. The tannin content is 16.26% w/w.

### Example 2

### Extraction of Salix branches and bark with a water-acetone solution (step a):

In this step, the total extract which can be used as the starting material for the subsequent column chromatography separation is prepared.

1000 grams of *Salix* branches and bark are extracted with 1.5 liters of 80% v/v acetone at 20°C for 4 hours in a static percolator. After 4 hours, the percolate is recovered and extracted 6 times again under the same conditions, but using 1 liter of solvent per extraction, to obtain approx. 7 liters of total percolate. The combined percolates are hot filtered and concentrated by a rotary evaporator at 60°C under reduced pressure. This extract has a total dry residue of 143 grams, the yield vs. starting material being 14.3% w/w.

The free salicin HPLC content is 4.3%; the total salicin HPLC content is 15.7% w/w. The tannin content is 15.42% w/w.

### Example 3

### Extraction of Salix branches and bark with water (step a):

In this step, the total extract which can be used as the starting material for the subsequent column chromatography separation is prepared.

1000 grams of *Salix* small branches and bark are covered with 1.5 liters of water at 20°C for 4 hours in a static percolator. After 4 hours, the percolate is recovered and extracted 6 times again under the same conditions, but using 1 liter of solvent per extraction, to obtain approx. 7 liters of total percolate. The combined percolates are filtered with suction and concentrated by a rotary evaporator at 60°C under reduced pressure. This extract has a total dry residue of 167 grams, the yield vs. starting material being 16.7% w/w.

The free salicin HPLC content is 3.94%; the total salicin HPLC content is 13.6% w/w. The tannin content is 6.8% w/w.

### Example 4

### Extraction of Salix branches and bark with methanol (step a):

In this step, the total extract which can be used as the starting material for the subsequent column chromatography separation is prepared.

1000 grams of *Salix* branches are covered with 1.5 liters of methanol at 20°C for 4 hours in a static percolator. After 4 hours, the percolate is recovered and extracted 6 times again under the same conditions, but using 1 liter of solvent per extraction, to obtain approx. 7 liters of total percolate. The combined percolates are filtered and concentrated by a rotary evaporator at 60°C under reduced pressure. This extract has a total dry residue of 101 grams, the yield vs. starting material being 10.1% w/w.

The free salicin HPLC content is 5,9%; the total salicin HPLC content is 19.9% w/w. The tannin content is 14.5% w/w.

### Example 5

### Purification of the extract of Salix branches and bark (step b): removal of water-insolubles:

Solution 1 obtained at the end of the workup described in Example 1 (step a) is concentrated by a rotary evaporator at 60°C under reduced pressure, to obtain an aqueous suspension with a dry residue of 25% w/w of the total aqueous suspension, the total weight of said solution being 615 g.

The resulting aqueous suspension is cooled at 4°C and left to stand for 16 hours, then the still cold aqueous suspension is centrifuged at 3000 g for 20 minutes to separate the precipitated residue from the clear aqueous solution. This precipitate, having a dry residue of 16.3 g, is rich in tannins and high molecular products and is removed.

The resulting clear solution (solution 2) has a dry residue equivalent to 137 g of partially purified extract having HPLC content in free salicin of 5.0% and HPLC content in total salicin of 16.7% w/w. The tannin content is 6.9% w/w.

The weight yield vs. starting material is 13.7% w/w.

### Example 6

### Purification of the extract of Salix branches and bark (step c):

The clear aqueous solution obtained at the end of the partial purification process of step b (Example 5, solution 2), having a dry residue of 137 g, is treated to remove water-soluble tannins.

The solution is added with 14 g of PVPP, corresponding to approx. 10% w/w. of the dry residue of the extract to treat. After stirring for 1 hour at room temperature, PVPP is separated from the solution by centrifugation.

The resulting solution (solution 3) has a dry residue equivalent to 125 g of partially purified extract, having HPLC content in free salicin of 5.3% and HPLC content in total salicin of 8% w/w. The tannin content is 1.2% w/w.

The weight yield vs. starting material is 12.5% w/w.

### Example 7

### Chromatographic purification of the extract of Salix branches and bark (step d):

The aqueous solution obtained from step c (Example 6, solution 3) is loaded onto a chromatographic column containing 1250 ml of Rohm and Haas XAD1180^{®} resin conditioned with water. The water-alcohol solution is adsorbed to the resin, while the unretained solution exiting the column is discarded. The resin is then washed with 1.25 liters of water, removing also this solution as its content in desired components is negligible. These discarded aqueous solutions (product 1) have in fact a total dry residue of 52.6 g, with HPLC content in free salicin of 0.87%, and HPLC content in total salicin of 0.92% w/w.

The column is eluted with 3.75 liters of 90% v/v aqueous ethanol. The resulting eluate is recovered and dried at 60°C under reduced pressure, to yield 72.4 grams of dry product (product 2), corresponding to a yield vs. the starting material of 7.2% w/w. HPLC content in free salicin is 8.95%, total salicin HPLC content is 30.0% w/w. The content in oligomeric proanthocyanidins is 11.2% w/w, the content in tannins is 2.1 % w/w.

### Example 8

### Chromatographic purification of the extract of Salix branches and bark (step d) and separation of free salicin from its derivatives:

The aqueous solution from step c (Example 6, solution 3) is loaded onto a chromatographic column containing 1250 ml of Rohm and Haas XAD1180^{®} resin conditioned with water. The water-alcohol solution is adsorbed to the resin, while the unretained solution exiting the column is discarded. The resin is then washed with 1.25 liters of water, removing also this solution as its content in desired components is negligible. These discarded aqueous solutions (product 3) have in fact a total dry residue of 51.7 g, with free salicin HPLC content of 1.34%, and total salicin HPLC content of 1.41 % w/w.

The resin is washed with 2.5 liters of 10% v/v aqueous ethanol, to obtain a solution (product 4) with a dry residue of 17.1 g (yield vs. starting material of 1.7% w/w.). Free salicin HPLC content is 34.6%, total salicin HPLC content is 34.9% w/w.

The column is eluted with 3.75 liters of 90% v/v aqueous ethanol. The resulting eluate is recovered and dried at 60°C under reduced pressure, to yield 43.2 grams of dry product (product 5), corresponding to a weight yield vs. starting material of 4.3% w/w, HPLC content in free salicin of 0.45%, HPLC content in total salicin of 39.7% w/w.

### Example 9

### Formulation of the extract in soft-gelatin capsules.

Formulation of *Salix rubra* extract in oily suspension for soft-gelatin capsules.

| **Unit composition:** | |
|---|---|
| *Salix rubra* extract according to Example 7 | 250 mg |
| Gliceryl monostearate | 30 mg |
| Soy lecithin | 10 mg |
| *Enothera biennis* oil q.s. to | 700 mg |

### Preparation:

1) Heat *Enothera biennis* oil at about 70°C and melt glyceryl monostearate therein, under stirring.
2) Add soy lecithin to the resulting solution.
3) Disperse *Salix rubra* extract in the resulting solution, promoting the homogeneous distribution with a suitable stirring system.

Gradually cool the resulting solution under stirring.

## Claims

1. A process for extracting salicin derivatives from *Salix* spp branches and bark, consisting in the sequence of steps a) to d):
a) Extraction of *Salix* spp branches and bark with suitable solvents which solubilize the desired products (total extract);
b) Removal of water-insoluble (or poorly soluble) tannins;
c) Optional removal of water-soluble tannins;
d) Purification on an adsorption resin column to increase the content of salicin derivatives.

2. A process as claimed in claim 1, in which step (a) is carried out by extraction of the vegetable material with a C1-C3 alcohol or mixtures of these solvents or aqueous solutions of these solvents or water alone.

3. A process as claimed in claim 2, in which the extraction solvent is ethanol.

4. A process as claimed in claim 2, in which the extraction solvent is a 30% water-ethanol solution in alcohol v/v.

5. A process as claimed in claim 2, in which the extraction solvent is acetone.

6. A process as claimed in claim 2, in which the extraction solvent is an 80% v/v water-acetone solution.

7. A process as claimed in claims 2-6, in which extraction is carried out at a temperature of 25°C.

8. A process as claimed in any one of claims 1 to 7, in which step (c) is carried out using polyvinylpolypyrrolidone (PVPP).

9. A process as claimed in any one of claims 1 to 7, in which the chromatographic separation is carried out on a styrene-divinyl benzene or acrylic resin.

10. A method to prepare medicaments for the treatment of arthrosis, rheumatoid arthritis and chronic-degenerative pathologies of joints, comprising the process of claim 1.

## Patentansprüche

1. Verfahren zum Extrahieren von Salicin-Derivaten aus Zweigen und Rinde von Salix spp., das aus der Folge von Schritten a) bis d) besteht:
a) Extraktion von Zweigen und Rinde von Salix spp. mit geeigneten Lösungsmitteln, die die gewünschten Produkte (Gesamt-Extrakt) solubilisieren;
b) Entfernung von in Wasser unlösliche (oder schlecht löslichen) Tanninen;
c) gegebenenfalls Entfernung von in Wasser löslichen Tanninen;
d) Reinigung an einer Adsorptions-Harz-Säule, um den Anteil an Salicin-Derivaten zu erhöhen.

2. Verfahren nach Anspruch 1, wobei Schritt (a) durch Extraktion von dem Pflanzen-Material mit einem C₁-C₃-Alkohol oder Gemischen von diesen Lösungsmitteln oder wässrigen Lösungen von diesen Lösungsmitteln oder nur Wasser ausgeführt wird.

3. Verfahren nach Anspruch 2, wobei das Extraktions-Lösungsmittel Ethanol ist.

4. Verfahren nach Anspruch 2, wobei das Extraktions-Lösungsmittel eine 30 %-ige Wasser-Ethanol-Lösung in Alkohol Volumen / Volumen ist.

5. Verfahren nach Anspruch 2, wobei das Extraktions-Lösungsmittel Aceton ist.

6. Verfahren nach Anspruch 2, wobei das Extraktions-Lösungsmittel eine 80 %-ige Volumen / Volumen Wasser-Aceton-Lösung ist.

7. Verfahren nach Ansprüchen 2-6, wobei eine Extraktion bei einer Temperatur von 25°C ausgeführt wird.

8. Verfahren nach einem der Ansprüche 1 bis 7, wobei Schritt (c) unter Verwendung von Polyvinyl-polypyrrolidon (PVPP) ausgeführt wird.

9. Verfahren nach einem der Ansprüche 1 bis 7, wobei die chromatographische Trennung an einem Styrol-Divinyl-benzol- oder Acryl-Harz ausgeführt wird.

10. Verfahren zum Herstellen von Medikamenten für die Behandlung von Arthrose, rheumatischer Arthritis und chronischdegenerativen Erkrankungen von Gelenken, umfassend das Verfahren nach Anspruch 1.

## Revendications

1. Procédé d'extraction de dérivés de salicine à partir de branches et d'écorce de *Salix* spp, consistant en la séquence d'étapes a) à d) :
a) extraction de branches et d'écorce de *Salix* spp avec des solvants appropriés qui solubilisent les produits désirés (extrait total) ;
b) élimination des tanins insolubles (ou faiblement solubles) dans l'eau ;
c) élimination facultative des tanins solubles dans l'eau ;
d) purification sur une colonne de résine d'adsorption pour augmenter la teneur en dérivés de salicine.

2. Procédé selon la revendication 1, dans lequel l'étape (a) est exécutée par extraction de la matière végétale avec un alcool en C₁-C₃ ou des mélanges de ces solvants ou des solutions aqueuses de ces solvants ou de l'eau seule.

3. Procédé selon la revendication 2, dans lequel le solvant d'extraction est l'éthanol.

4. Procédé selon la revendication 2, dans lequel le solvant d'extraction est une solution à 30% eau-éthanol dans un alcool en volume.

5. Procédé selon la revendication 2, dans lequel le solvant d'extraction est l'acétone.

6. Procédé selon la revendication 2, dans lequel le solvant d'extraction est une solution à 80% en volume eau-acétone.

7. Procédé selon les revendications 2 à 6, dans lequel l'extraction est exécutée à une température de 25°C.

8. Procédé selon l'une quelconque des revendications 1 à 7, dans lequel l'étape (c) est exécutée en utilisant une polyvinylpolypyrrolidone (PVPP).

9. Procédé selon l'une quelconque des revendications 1 à 7, dans lequel la séparation chromatographique est exécutée sur une résine de styrènedivinylbenzène ou acrylique.

10. Procédé de préparation de médicaments pour le traitement de l'arthrose, de l'arthrite rhumatoïde et de pathologies chroniques dégénératives des articulations, comprenant le procédé selon la revendication 1.
